# EUROPÄISCHE PATENTANMELDUNG

(11) **EP 2 130 437 A1**
(43) Veröffentlichungstag der Anmeldung: **09.12.2009**
(21) Anmeldenummer: 08157488.1
(22) Anmeldetag: 03.06.2008
(51) Int. Cl.: A21D 8/04, C12N 1/18

(54) **Einstufige Backwarenherstellung**

(71) Anmelder: Ernst Böcker GmbH & Co. KG, 32427 Minden (DE)
(72) Erfinder: Brandt, Markus, 32423, Minden (DE); Böcker, Georg, 32427, Minden (DE)
(74) Vertreter: Fleuchaus, Andrea

(57) **Zusammenfassung**

Die vorliegende Erfindung betrifft ein Verfahren zur Herstellung von Backwaren aus Sauerteig. Weiterhin betrifft die vorliegende Erfindung die Zusammensetzung eines Triebferments zur verbesserten, beschleunigten und erleichterten, gleichzeitig aber qualitätsstabilen Herstellung von Sauerteigen und den daraus resultierenden Backwaren.

## Beschreibung

Die vorliegende Erfindung betrifft ein Verfahren zur Herstellung von Backwaren aus Sauerteig. Weiterhin betrifft die vorliegende Erfindung die Zusammensetzung eines Triebferments zur verbesserten, beschleunigten und erleichterten, gleichzeitig aber qualitätsstabilen Herstellung von Sauerteigen und den daraus resultierenden Backwaren.

Das Mischen von Getreidemahlerzeugnissen und Wasser führt nach einer gewissen Abstehzeit unweigerlich zu einem Sauerteig, der durch seinen sauren Geschmack, seine Fermentationsaromen und eine Volumenerhöhung auf Grund mikrobieller Gasbildung charakterisiert ist. Diese sogenannte Getreidefermentation wird dabei in der Regel von gleichzeitigem oder aufeinander folgendem Wachstum der im Mehl befindlichen Milchsäurebakterien und Hefen verursacht. Andere Keimgruppen werden mit Beginn der Gärung durch die anaeroben Bedingungen und die Säuerung des Teig auf pH-Werte zwischen 3 und 4 gehemmt.

Der Begriff Milchsäurebakterien ist ein historisch entstandener Begriff für eine Gruppe von Bakterien, welche als gemeinsame physiologische Eigenschaft Milchsäure als ein Hauptprodukt des Kohlenhydratstoffwechsels bilden. Milchsäurebakterien sind Gram-positive, anaerobe oder fakultativ anaerobe, nicht-Sporen bildende Kokken oder Stäbchen. Ein besonderes Kennzeichen ist ihr eingeschränktes Potential zur Biosynthese von Zellbestandteilen z.B. Vitaminen, Aminosäuren, Purinen und Pyrimidinen. Sie werden nach derzeit gängiger Taxonomie als die Gattung der *Lactobacillaceae* bezeichnet. Heute werden auf Grund der verbesserten, molekular biologischer Methoden der Taxonomie weitere fünfzehn Gattungen den Milchsäurebakterien zugeordnet, zu diesen gehören die für die Lebensmittelerzeugung relevanten Gattungen *Carnobacterium, Enterococcus, Lactobacillus, Lactococcus, Leuconostoc, Oenococcus, Pediococcus, Streptococcus, Tetragenococcus* und *Weissella*.

Da die Qualität aller fermentierten Lebensmittel entscheidend von der Zusammensetzung der Fermentationsflora abhängt, werden die wichtigsten Leitkeime aus der Familie der Milchsäurebakterien, die bei der Sauerteigfermentation vorkommen, in der nachfolgenden Tabelle 1 aufgelistet.

**Tabelle 1:**

| **Homofermentativ** | **Heterofermentativ** | **Morphologie** |
|---|---|---|
| *L. salivarius* | *L. sanfranciscensis* | Stäbchen |
| *L. mindensis* | *L. fermentum* | Stäbchen |
| *L. casei* | *L. cellobiosus* | Stäbchen |
| *L. coryniformis* | *L. brevis* | Stäbchen |
| *L. curvatus* | *L. pontis* | Stäbchen |
| | *L. hammesii* | Stäbchen |
| | *L. brevis* | Stäbchen |
| *L. plantarum* | *L. panis* | Stäbchen |
| *E. faecalis* | *Le. paramesenteorides* | Kokken |
| *Lc. lactis* | *W. cibaria* | Kokken |
| *P. parvulus* | | Kokken |
| *P. pentosaceus* | | Kokken |

Eine besonders an das Wachstum in Sauerteig angepasste Milchsäurebakterienart ist *Lactobacillus sanfranciscensis* Sie zeichnet sich z.B. durch ihre Fähigkeit aus sehr schnell anzusäuern und sich schnell an verändernde Umgebungsbedingungen anpassen zu können. Ihr effektiver Maltosestoffwechsel, die Fähigkeit die in Sauerteigen vorhandenen Elektronenakzeptoren zur ATP-Bildung nutzen zu können, sowie das an Getreidesubstrat angepasste Stoffwechselverhalten, fördern die Dominanz dieses heterofermentativen *Lactobacillus* insbesondere in kontinuierlich geführtem Sauerteig.

Obwohl eine Vielzahl von Milchsäurebakterien aus Sauerteigen isoliert wurde, finden sich trotzdem in einem Sauerteig in der Regel nicht mehr als 1 bis 4 unterschiedliche Stämme von Milchsäurebakterien und 1 bis 2 unterschiedliche Stämme von Hefen, die derselben oder unterschiedlich Arten angehören können. Ein Erklärungsmodell ist hierfür, dass die Mikroorganismen, die in der Lage sind sich schnell an sich verändernde Umgebungsbedingungen anpassen zu können, einen Wachstumsvorteil haben.

Die verschiedenen, in Weizen- und Roggensauerteigen auftretenden Hefen haben dabei normalerweise einen Anteil von weniger als 0,1 bis 10% der Gesamtflora. In Tabelle 2 sind die in Sauerteigen häufig vorkommenden Hefen aufgelistet.

**Tabelle 2:**

| **Leitkeime** | **Häufige Isolate** |
|---|---|
| *C. humilis* | *S. pastorianus* |
| *C. milleri* | *S. minor* |
| *S. exiguus* | *S. fruetum* |
| *S. cerevisiae* | *C. holmii* |
| | *C. krusei* |

Typischerweise kommen Hefen in Keimzahlen bis 9 x 10⁴KbE/g im Getreide und bis 2 x 10³KbE/g im Mehl vor. Sie können auch die Ursache für Fehlgärungen im Sauerteig sein. In Getreide deutscher Herkunft finden man typischerweise 14 verschiedene Hefespezies, darunter die Gattungen *Candida, Cryptococcus, Pichia, Rhodotorula, Trichosporon, Saccharomyces, Sporobolomyces*.

Es sei angemerkt, dass nicht alle diese Hefespezies erwünscht sind. So ist z.B. *Trichosporon cutaneum* eine häufig vertretene Hefe auf Getreide und dessen Mahlerzeugnissen. Stämme dieser Hefe, ebenso wie die in geringerer Zahl gefundenen, *Candida zeylanoides* und *Sporobolomyces salmanicolor*, sind aber potentiell humanpathogen.

Grundsätzlich enthalten reife Sauerteige zwischen 5 x 10⁸ und 5 x 10⁹KbE/g Milchsäurebakterien und zwischen 10³ und 10⁸ KbE/g Hefen. Bei Keimzahlen von weniger als 10⁷ Milchsäurebakterien/g bzw. weniger als 10⁵ Hefen/g im reifen Sauerteig kann nicht von einem relevanten Beitrag der Organismen zum Stoffumsatz ausgegangen werden.

Brot und andere Backwaren, die mit Hilfe von Sauerteigen hergestellt werden, zeichnen sich durch ihre besondere Qualität aus. Während bei Roggenmahlerzeugnissen eine Teigsäuerung erforderlich ist, um die Backfähigkeit zu gewährleisten, dient die Sauerteigführung bei Weizen vorrangig der Verbesserung der sensorischen Qualität, insbesondere des Aromas, die notwendige Triebleistung wird heutzutage durch zugesetzte Backhefe erbracht.

Mit Hilfe von Sauerteig hergestellte Brote zeichnen sich durch ihr charakteristisches Aroma, verbesserte Frischhaltung und längere mikrobiologische Stabilität aus. Diese Qualität wird maßgeblich von den Stoffwechselleistungen der Fermentationsflora beeinflusst. Einen entscheidenden Einfluss hat außerdem die Fermentationsdauer, bzw. haben die herrschenden Prozessbedingungen während der Sauerteigherstellung.

Bei der Sauerteigherstellung unterscheidet man zwischen Spontansauerteigen und beimpf ten Sauerteigen.

Spontansauerteige werden durch Mischen von Mehl und Wasser ohne Zusatz von Anstellgut oder Starterkultur hergestellt. Die Mikroflora von Spontansauerteigen wird in erster Linie durch die Mikroflora des Mehles geprägt und kann je nach Art und Herkunft des Getreideproduktes stark variieren. Wenn ein aus Mehl und Wasser hergestellter Spontansauerteig als Anstellgut für einen geführten Sauerteig verwendet wird, stellt sich nach wenigen Führungsstufen eine für die jeweilige Führungsparameter charakteristische Fermentationsflora ein, die mit der Mikroflora des Getreides nicht mehr im Zusammenhang steht.

Die Untersuchung der charakteristischen Florententwicklung in Spontansauerteigen wird seit langem wissenschaftlich dokumentiert. Hochstrasser *et al.* (1993, Mitt. Gebiete Lebensm. Hyg. 84: 356-381) dokumentiert und berichtet z.B. über die Keimzahlen von Enterobakterien und Milchsäurebakterien im Mehl, die zunächst unterhalb von 10³KbE/g liegen, sowie deren Veränderungen im Laufe einer Sauerteigführung. Hierbei wurde gezeigt, dass nach der ersten Stufe die Fermentationsflora vor allem durch Enterobakterien dominiert wird. Bereits nach einer weiteren Stufe überstieg die Keimzahl von Milchsäurebakterien die der Enterobakterien um den Faktor 100, da letztere durch die tiefen pH-Werte gehemmt werden. Nach vier Führungsstufen ist die Gesamtkeimzahl praktisch identisch zur Keimzahl der Milchsäurebakterien, zudem baut sich zunehmend eine Population von Hefen auf.

In diesem Zusammenhang mit Spontansauerteigen ist es wichtig die Notwendigkeit der mehrstufigen Teigführung hervorzuheben, denn sie hat einen entscheidenden Einfluss auf die Zusammensetzung der Mikroflora im Teig. Denn es ist zu bedenken, dass bei zu kurzer Führung oder direkter Führung das Risiko einer Enterobakterienkontamination bestehen bleibt. Ferner finden sich bei zu kurzer Führung noch nicht ausreichend Hefen im Teig, um die gewünschte Volumenvergrößerung und damit Teigausbeute zu erreichen. Auch die Geschmacksentwicklung durch Stoffwechselprodukte der Milchsäurebakterien ist ebenfalls noch dürftig.

Ein weiterer Nachteil von Spontansauerteigen ist es, dass die Zusammensetzung der Mikroflora in Spontansauerteigen erheblich von der Mikroflora der Rohware und deren Kontaminationen abhängt. Es muss daher mit erheblich größeren Schwankungen und insbesondere starken Qualitätsschwankungen bei den aus Spontansauerteigen hergestellten Backwaren gerechnet werden.

Um die Mikroflora von Sauerteigen zu beeinflussen werden Sauerteige - im Unterschied zu Spontansauerteigen - mit einer Starterkultur bzw. Anstellgut beimpft. Durch die Verwendung von Anstellgut können Fehlgärungen vermieden werden und bei gleich bleibender Mikroflora kann eine standardisierte Brotqualität erreicht werden.

Da die Keimzahlen von Milchsäurebakterien und Hefen im Anstellgut etwa 10- bis 1000-fach höher sind als die Keimzahl in der Rohware, spielt der Keimbesatz des Mehles für die Mikroflorenentwicklung praktisch keine Rolle mehr.

Bei der Herstellung von beimpften Sauerteigen unterscheidet man zunächst zwischen der direkten und der indirekten Teigführung.

Unter indirekter Teigführung versteht man allgemein die Prozessbedingungen zu einer klassischen Heranführung des Sauerteiges in mindestens 3 Stufen, gerne auch bis zu 9 Stufen. Entscheidend ist, dass bei der indirekten Führung ein gezieltes Anzüchten bzw. eine gezielter Einsatz von Mikroorganismen in den Vorstufen stattfindet. In Tabelle 3 sind die üblichen Bezeichnungen dieser Stufen, am Beispiel für Roggen, sowie typische Prozessbedingungen aufgeführt.

**Tabelle 3:**

| | **Stufe 1 -Anfrischsauer** | **Stufe 2 -Grundsauer** | **Stufe 3 - Vollsauer** |
|---|---|---|---|
| Reifezeit | 5 - 8 Std. | 6 - 10 Std. | 3 - 10 Std. |
| Temperatur | 25 - 26 °C | 23 - 28 °C | 25 - 32 °C |
| Vorgang | Hefevermehrung | Säure- und Aromabildung | Optimierung der Gärleistung und Säurebildung |

In Tabelle 4 sind ferner diese Stufen, am Beispiel für Weizensauerteig z.B. für eine Panettoneherstellung, aufgeführt. Die klassische Panettoneherstellung wird in 2 bis 3 weiteren aufwendigen Teigstufen zur fertigen Panettone geführt.

**Tabelle 4:**

| | **Stufe 1** | **Stufe 2** | **Stufe 3** |
|---|---|---|---|
| Reifezeit | 2 - 8 Std. | 2 - 8 Std. | 2 - 8 Std. |
| Temperatur | 18 - 23 °C | 18 - 23 °C | 22 - 28 °C |
| Vorgang | Hefevermehrung | Hefevermehrung und Aromabildung | Optimierung der Gärleistung und Aromabildung |

In Tabelle 5 sind ferner die Stufen der Baguetteherstellung aufgeführt, wobei bekanntlich dieses Herstellungsverfahren mit seinen 3 bis 9 Stufen eines der aufwendigsten Brotsortenherstellungsverfahren ist.

**Tabelle 5:**

| | **Stufe 1** | **Stufe 2** | **Stufe 3** | **Stufe 4** | **Stufe 5** |
|---|---|---|---|---|---|
| Reifezeit | 10 - 16 Std. | 20 - 26 Std. | 16 - 20 Std. | 8-12 Std | 10-14 Std |
| Temperatur | 24 - 26 °C | 14 - 16 °C | 14 - 16 °C | 14-16 °C | 14-16 °c |

Die jeweiligen Reifezeiten und Temperaturangaben der vorstehenden Tabellen können variieren in Abhängigkeit der Mengenverhältnisse der Zutaten sowie in Abhängigkeit der eingesetzten Starterkulturen.

Unabhängig von den speziellen Prozessparametern ist jedoch erkennbar, dass eine indirekte Teigführung aufwendig und sehr zeitintensiv ist.

Die sogenannte direkte Führung verzichtet auf mehrstufige Vermehrungsverfahren und unterscheidet sich im Wesentlichen von der indirekten Führung dadurch, dass der mikrobielle Stoffwechsel praktisch ausschließlich auf die in hoher Keimzahl zugesetzten Backhefen zurückzuführen ist.

Vergleicht man die direkte und indirekte Führung so ist es als ein Nachteil der indirekten Führung zu beurteilen, dass dieses mehrstufige Führungsverfahren sehr zeitintensiv ist und handwerkliches Können erfordert. Gleichzeitig ist Schnelligkeit und leichte Handhabung als ein Vorteil der direkten und backhefehaltigen Führung zu betrachten. Derartige direkt geführte und backhefehaltige Teige zeichnen sich weiter durch Ihre gute Triebkraft und einfache Standardisierbarkeit aus. Allerdings fehlt den aus direkt geführten Teigen oder aus backhefehaltigen Teigen erzeugten Backwaren das typische und erwünschte Aroma. Ferner weisen solche Backwaren auch nur mäßig gute Frischhalteeigenschaften auf.

In traditionellen und industriellen Backverfahren spricht man weiterhin von drei Sauerteigtypen.

Typ I Sauerteige werden traditionell hergestellt und zeichnen sich dadurch aus, dass eine kontinuierliche, meist tägliche Weiterführung (Fütterung) nötig ist, um die Mikroorganismen in einen aktiven Stoffwechselzustand zu halten. Der mindestens dreistufige Fermentationsprozess der Typ I Sauerteige wird dabei gewöhnlich bei Temperaturen unter 30°C durchgeführt.

Typ II Sauerteige sind weniger aufwendig und zeichnen sich durch einen bis zu 5 Tagen dauernden einstufigen Fermentationsprozess aus. Der Fermentationsprozess der Typ II Sauerteige wird dabei gewöhnlich bei Temperaturen über 30°C durchgeführt. Im Typ II Sauerteig zeigen die meisten Mikroorganismen nur einen eingeschränkten Stoffwechsel. Diese Typ II Sauerteige werden zu meist in industriellen Verfahren eingesetzt und dienen als Geschmacksgeber bzw. zum Ansäuern; sie entwickeln selber zu wenig Triebleistung, um ausschließlich eingesetzt zu werden.

Unter Typ III Sauerteigen, versteht man getrocknete Fermentationsprodukte, die vornehmlich als Geschmacksgeber bzw. zum Ansäuern eingesetzt werden. Auch diese entwickeln selber zu wenig Triebleistung, um ausschließlich und ohne den Zusatz von Backhefe eingesetzt zu werden.

Es ist daher eine Aufgabe der Erfindung ein verkürztes und verbessertes Verfahren zur Sauerteigherstellung bereitzustellen, um die Vorteile eines indirekt geführten Teiges, nämlich gutes Aroma, verbesserte Frischhalteeigenschaft, mit den Vorteilen eines direkt geführten Teiges, nämlich schnelle und einfache Handhabung, zu kombinieren.

Ein wesentliches Augenmerk ist dabei auf die Tatsache zu richten, dass die Erfindung den Einsatz von Backhefe wie er in der direkten Führung üblicherweise eingesetzt wird, vermeidet und ablehnt, denn die angestrebte Aromaverbesserung in den Backwaren wird vornehmlich durch den Stoffwechsel von Milchsäurebakterien erreicht und beeinflusst.

In diesem Zusammenhang ist es wichtig zu definieren, dass im Rahmen dieser Erfindung mit dem Begriff"Backhefe", die auch als Bäckerhefe bezeichnet wird, solche Stämme von *Saccharomyces cerevisiae* gemeint sind, die speziell für den Einsatz zur Teigerzeugung kultiviert werden. Hierzu wird *S*. *cerevisiae* auf Melasse und unter Zusatz von Nährsalzen kultiviert. Dies führt dazu, dass die auf Melasse gezogenen Stämme bevorzugt Saccharose verwerten und nicht mehr die im Teig in großer Menge zur Verfügung stehende Maltose verarbeitet. Im Gegensatz hierzu sind die für die Sauerteigerzeugung geeigneten Hefen auf Getreide gezogen. Sie sind also an Maltose und Glucose adaptiert und damit in der Lage diese auch effizient zu verarbeiten.

Neben der Tatsache, dass die Hefezuchtverfahren kompliziert sind, hat die Stoffwechselanpassung der auf Melasse gezüchteten Hefe zur Konsequenz, dass die Stämme vom Bäcker selber nicht in ausreichender Qualität regeneriert werden können und damit eine erhebliche Abhängigkeit sowie ein erheblicher Kostenfaktor entsteht.

Durch das Triebferment und das Verfahren der vorliegenden Erfindung, kann der Bäcker auf den Einsatz von Backhefe verzichten und realisiert somit eine erhebliche finanzielle Einsparung. Ferner macht er sich unabhängig von der Verfügbarkeit frischer und einsatzbereiter Backhefe.

In ihrer breitesten Ausführungsform stellt die vorliegende Erfindung daher eine neue Zusammensetzung eines Triebferments sowie ein Verfahren zur backhefefreien Erzeugung von Sauerteigen und Backwaren bereit.

Das Triebferment enthält eine Mischung aus wenigstens zwei oder mehreren kultivierten Reinkulturen von Milchsäurebakterien, wobei wenigstens eine der kultivierten Reinkulturen ein Stamm aus der Familie der Pediokokken und/oder Weissella ist. Weiterhin enthält das Triebferment wenigstens einen kultivierten Stamm eines Hefesprosspilzes. Das Triebferment enthält gemäß der vorliegenden Erfindung keine Backhefe und/oder keinen auf Melasse gezüchteten oder kultivierten Stamm eines Hefesprosspilzes.

Die im Triebferment enthaltenen kultivierten Reinkulturen sind ausgewählt aus der Gruppe der Milchsäurebakterien, welche beispielsweise die Stämme *L. plantarum, L. pontis, L. sanfranciscensis, L. crispatus, L. suntoryeus, Le. argentinum, L. helveticus, L. paralimentarius, L. fermentum, L. paracasei, L. frumenti, L. alimentarius, W cibaria, W confusa, P. acidilactici, P. parvulus* und *P. pentosaceus* enthält. Erfindungsgemäß ist eine Keimdichte der Milchsäurebakterien von 1 x 10⁷ bis 2 x 10⁹KbE/g erwünscht.

Insbesondere enthält die Zusammensetzung des Triebferments gemäß der Erfindung wenigstens einen Pediokokken oder Weisella Stamm. Die im Triebferment enthaltenen Stämme sind dabei ausgewählt aus der Gruppe, welche die Stämme *P. acidilactici, P. parvulus, P. pentosaceus,* sowie *W. cibaria* und *W. confusa* enthält.

Der Zusatz von einer Reinkultur an Pediokokken und/oder Weissella ist dabei sehr ungewöhnlich, da Pediokokken und Weissella bislang nur als Kontamination und daher als untauglich für die Teigfermentation betrachtet wurden. Gemäß eines Ausführungsbeispieles enthält das Triebferment 1x10⁶ bis 3 x 10⁹KbE/g eines Pediokokken- oder Weissella-Stammes bezogen auf die Gesamtkeimzahl der Mikroorganismen in der Trockenmasse des Triebferments.

Pediokokken sind homofermentativ und säuern verhältnismäßig langsam und auch nur mild an. Erstaunlicherweise konnte dennoch im Rahmen der Erfindung gezeigt werden, dass bei der kurzen, direkten Teigführung gemäß dem Verfahren der vorliegenden Erfindung die milde Ansäuerung durch Pediokokken bzw. auch Weissella Stämme, welche durch eine langsamere Veränderung der Bedingungen im Teig eine ideale CO2-Produktionsrate ermöglichen, ausreicht, um eine erstklassige Teigherstellung zu ermöglichen.

Die Erfindung konnte daher zeigen, dass überraschenderweise der Zusatz von Pediokokken bzw. Weissella zum Triebferment eine unerwartet kurze und damit einstufige Teigführung ermöglicht und der dabei erhaltene Sauerteig gute Triebleistung, ideale (nämlich leichte) Säuerung und gutes bzw. mildes Aroma aufweist.

Durch das erfindungsgemäße Triebferment konnte daher die Zeitdauer zur Herstellung eines reifen Sauerteigs im einstufigen Verfahren auf minimal 5 Stunden, gemäß anderer Ausführungsbeispiele 8 Stunden, gemäß anderer Ausführungsbeispiele 10 Stunden, gemäß anderer Ausführungsbeispiele 12 Stunden bis 14 Stunden reduziert werden.

Weiterhin enthält das Triebferment der vorliegenden Erfindung wenigstens einen kultivierten Stamm eines Hefesprosspilzes, ausgewählt aus der Gruppe bestehend aus *C. humilis, C. milleri, S. exiguus, T. delbrueckii, S. minor, S. pastorianus, S. cerevisiae* und *S*. *fructuum*, wobei die besagten Hefesprosspilze zu keinem Zeitpunkt auf Melasse gezüchtet oder kultiviert wurden, denn durch eine Kultivierung auf Melasse verändern sich die Stoffwechselwege und damit die geschmacksgebenden Eigenschaften der Stämme. Erfindungsgemäß ist eine Keimdichte der Hefesprosspilze im Triebferment von 1 x 10⁵ bis 5 x 10⁸KbE/g erwünscht.

Weiterhin bevorzugt die erfindungsgemäße Auswahl solche Stämme der aufgeführten Hefesprosspilze die säure-adaptiert sind, und damit während der Sauerteigfermentation nicht verdrängt werden. Dem Fachmann sind zahlreiche solche Stämme bekannt, im Zweifelsfall können solche Stämme durch die Kultivierung von Hefen auf Getreide jedoch nicht auf Melasse gezogen werden. Diese Getreidekultur ist langwieriger aber sie führt zu Hefesprosspilzen, die ihren Stoffwechsel angepasst haben und Maltose an Stelle von Saccharose verstoffwechseln, gleichzeitig entwickeln sich bei solchen Getreidekulturen bevorzugt solche Hefesprosspilze, die an Säuren z.B. Milch- und Essigsäuren, die während der Kultivierung auf Getreide entstehen, adaptiert sind. Sie werden daher als "säure-adaptiert" bezeichnet.

Das Triebferment der vorliegenden Erfindung ermöglicht es dabei einen Vorteig oder Sauerteig in direkter Führung zu erzeugen, der nach einer Inkubation von 3 bis 12 Stunden bei Temperaturen zwischen 16° und 30° C einen Milchsäuregehalt von 0,5 %, vorzugsweise 0,3% und maximal 1 % aufweist.

Gemäß einer weiteren Ausführungsform stellt die vorliegende Erfindung daher auch ein Verfahren zur verbesserten direkten Führung eines Sauerteiges bereit.

Die direkte Führung bedeutet im Rahmen der vorliegenden Erfindung, dass in einem ersten Schritt Mehl mit Wasser und dem Triebferment angesetzt wird und diese Mischung für 3 bis 24 Stunden, vorzugsweise 3 bis 6 Std., weiter vorzugsweise 4 bis 8 Std., weiter vorzugsweise 4 bis 12 Std., weiter vorzugsweise 6 bis 12 Std., weiter vorzugsweise 6 bis 18 Std. und weiter vorzugsweise 5 bis 24 Std. inkubiert wird.

Die Inkubationstemperatur liegt dabei zwischen 15° und 30°C, vorzugsweise zwischen 15° und 20°C, weiter vorzugsweise zwischen 18° und 22°C, weiter vorzugsweise zwischen 18° und 24°C, weiter vorzugsweise zwischen 18° und 26°C, weiter vorzugsweise zwischen 20° und 24°C, weiter vorzugsweise zwischen 21° und 26°C. Situationsbedingt kann die Temperaturkurve auch ansteigend oder abfallend gewählt werden.

Nach der einstufigen Teigfermentation werden oder können weitere Backzutaten z.B. Zucker, Mehl, Eier, Mandeln, Früchte und/oder Aromastoffe dem Teig zugegeben werden. Die Zugabe von Triebmittel wie Backhefe oder Backpulver sind nicht nötig und wird erfindungsgemäß abgelehnt.

Das erfindungsgemäße Verfahren sieht weiter vor, dass nach einer 0,5 bis 6 stündigen, vorzugsweise 1 bis 4 stündigen, weiter vorzugsweise 1 bis 3 stündigen Ruhe- bzw. Gärphase die Backware ausgebacken werden kann.

Entscheidend für die Bewertung der Qualität der Backwaren, die entsprechend den erfindungsgemäßen Verfahren hergestellt wurden, ist es dabei, dass sie einen Milchsäuregehalt von 0,5 %, vorzugsweise 0,4 %, weiter vorzugsweise 0,3 % und maximal 1,0 % aufweisen.

Der Milchsäuregehalt des Teigen vor dem Ausbacken bzw. der fertigen Backwaren kann dabei mittels dem HPLC Verfahren, das dem Fachmann wohlbekannt ist, ermittelt werden.

Ein weiterer Parameter zur Bestimmung der Teigqualität ist der Gärungsquotient, der das molare Verhältnis von Milch- zu Essigsäure angibt. Essigsäure beeinflusst den Geruch, Geschmack und die Haltbarkeit von Backwaren wesentlich stärker als Milchsäure. Es ist daher ein Ziel der Erfindung, durch die Wahl und den Zusatz von heterofermentativen Milchsäurebakterien zum Triebferment, den Gärungsquotient so zu beeinflussen, dass gemäß einer Attsführungsform in Roggensauerteigen, die mit dem Triebferment der vorliegenden Erfindung bzw. nach dem Verfahren der vorliegenden Erfindung hergestellt werden, nach einer Inkubationszeit von wenigstens 5 Stunden, vorzugsweise 9, vorzugsweise 10, vorzugsweise 12 Stunden und maximal 16 Stunden der Gärungsquotient 1,7 bis 2,8, vorzugsweise 2,3 bis 3,0, weiter vorzugsweise 2,5 bis 3,0 und weiter vorzugsweise 3,0 bis 3,5 beträgt.

Gemäß einer weiteren Attsführungsform wird mit dem Verfahren der vorliegenden Erfindung bzw. unter Einsatz des Triebferments der vorliegenden Erfindung ein Weizenvorteig bzw. Weizensauerteig erzeugt, der nach einer Inkubationszeit von wenigstens 5 Stunden, vorzugsweise 9, vorzugsweise 10, vorzugsweise 12 Stunden und maximal 16 Stunden einen Gärungsquotient von 1,5 bis 10, vorzugsweise 2,3 bis 3,0, weiter vorzugsweise 2,5 bis 3,5, weiter vorzugsweise 3,0 bis 4,0, weiter vorzugsweise 3,8 bis 5,0, weiter vorzugsweise 4,0 bis 6,0, weiter vorzugsweise 5,5 bis 7,0, weiter vorzugsweise 6,0 bis 8,0, weiter vorzugsweise 6,5 bis 9,0, weiter vorzugsweise 7,0 bis 9,5 und weiter vorzugsweise von 7,8 bis 10 erreicht. Dieser Gärungsquotient wirkt sich dabei deutlich auf eine verbesserte Qualität der Backware und insbesondere ein verbessertes, feines und leicht saueres Brotaroma aus.

Der Gärungsquotient wird üblicherweise als Verhältnis der im Teig vorliegenden Mengen an Milch- bzw. Essigsäure mittels HPLC ermittelt.

Die Abbauleistung durch das Triebferment äußert sich auch durch den Anstieg der frei verfügbaren Aminosäuren, die dann zur Geschmacksbildung wesentlich beitragen können, durch Bildung von Zuckerestern, aber auch durch den natürlichen Abbau weiter zu Fuselalkoholen und Aldehyden.

Gemäß einer weiteren Ausführungsform wird mit dem Verfahren der vorliegenden Erfindung bzw. unter Einsatz des Triebferments der vorliegenden Erfindung ein Vorteig bzw. Sauerteig erzeugt, der nach einer Inkubationszeit von wenigstens 5 Stunden, vorzugsweise 8, vorzugsweise 10, vorzugsweise 12 Stunden und maximal 16 Stunden eine Gehalt an Leucin von 0.1- 6mg per kg Teig, vorzugsweise 0,5 - 4 mg/kg, weiter vorzugsweise 0,5 - 2 mg/kg, Isoleucin von 0,1 - 5 mg/kg Teig, vorzugsweise 0,5 - 3 mg/kg, weiter vorzugsweise 0,5 - 1,5 mg/kg, Methionin von 0,1 - 6 mg/kg Teig, vorzugsweise 0,5 - 4 mg/kg, weiter vorzugsweise 0,5 - 2 mg/kg, Valin von 0,1 - 6 mg/kg Teig, vorzugsweise 0,2 - 4 mg/kg, weiter vorzugsweise 0,3 - 2 mg/kg und/oder Phenylalanin von 0,1 - 4 mg/kg Teig, vorzugsweise 0,5 - 2 mg/kg, weiter vorzugsweise 0,5 - 1,5 mg/kg, weiter vorzugsweise 0,6 - 1 mg/kg hat. Der Aminosäuregehalt kann ebenfalls mittels der bekannten HPLC Technik analysiert werden.

Das Verfahren der vorliegenden Erfindung bzw. der Einsatz des Triebferments der vorliegenden Erfindung zur Herstellung eines Vorteiges bzw. Sauerteiges weist eine hervorragende Triebleistung auf. Diese Treibleistung ist unter anderem auf die CO₂ Produktionsrate der Mikroorganismen im Teig zurückzuführen. Gemäß einer weiteren Ausführungsform wird mit dem Verfahren der vorliegenden Erfindung ein Vorteig bzw. Sauerteig erzeugt, der nach einer Inkubationszeit von wenigstens 5 Stunden, vorzugsweise 9, vorzugsweise 10, vorzugsweise 12 Stunden und maximal 16 Stunden eine CO₂ Produktionsrate von 70 bis 300 ml/100g Mehl , vorzugsweise 70 bis 150, weiter vorzugsweise 120 bis 250 ml/ 100g Mehl aufweist.

Zur Bestimmung der CO₂ Produktionsrate wird das gebildete Gas aus dem Teig aufgefangen und volumetrisch durch Verdrängen einer gesättigten Kochsalzlösung gemäß AACC Methode 12-10 erfasst.

Das Verfahren der vorliegenden Erfindung bzw. der Einsatz des Triebferments der Erfindung zur Herstellung eines Vorteiges bzw. Sauerteiges bringt bei vermindertem Zeiteinsatz und bei direkter Teigführung ein hervorragendes Backprodukt bzw. Brot hervor, welches sich insbesondere durch sein feines, leicht saueres Aroma auszeichnet. Quantifizierbar ist dieses Aroma z.B. und gemäß einer weiteren Ausführungsform durch seinen Gehalt an Vanillin in der Krume des ausgebackenen Brotes, der größer als 1000 µg/kg, vorzugsweise größer als 1500 µg/kg, weiter vorzugsweise größer als 2000 µg/kg, weiter vorzugsweise größer als 2500 µg/kg Trockenmasse ist.

Das erfindungsgemäße Triebferment und Verfahren eignet sich zur Herstellung von Rog gensauerteigen, aber insbesondere auch zur Herstellung von Weizensauerteigen und den daraus erzeugten Backwaren. Durch die Wahl der Mikroorganismen und die erfindungsgemäße Prozessführung wird ein intensives Aroma bei milder Säuerung des Teiges erreicht.

Gleichzeitig wird durch die Wahl der Mikroorganismen zusammen mit der erfindungsgemäßen Prozessführung gewährleistet, dass die Mikroflora und das Verhältnis der Milchsäurebakterien zu den Hefen, nämlich z.B. den säureadaptierten Hefen, im Teig stabil bleibt. Dies ermöglicht, den erzeugten Sauerteig oder Weizensauerteig über mehrere Tage hin immer wieder als Anstellgut für erneute Sauerteige zu verwenden, ohne dass es dabei zu einer Verschiebung der Brotqualität sowie der Mikroflora des Teiges bzw. der Triebleistung des Teiges kommt. Idealerweise und um Qualitätsschwankungen zu vermeiden, wird dennoch einmal pro Woche ein neuer Teig mit frischem Triebferment gestartet.

Gemäß einer weiteren Ausführungsform haben Backwaren und insbesondere Brote, wie z.B. Weizenbrote, die mit dem Triebferment gemäß der vorliegenden Erfindung hergestellt wurden, einen leicht bestimmbaren aber charakteristischen Gehalt von 0,3 bis 1,8 % Maltose, vorzugsweise 0,7 bis 1,5 % Maltose, weiter vorzugsweise 0,8 bis 1,2 % Maltose.

Im Vergleich hierzu haben backhefehaltige Brote etwa 2,5 % Maltose. Die Maltosemenge kann mittels der dem Fachmann bekannten Technologie der HPLC-Messung bestimmt werden.

Die Erfindung wird nachfolgend anhand einiger Beispiele, die nicht limitierend sind und lediglich Anregungen für den Fachmann darstellen, näher erläutert.

### Beispiele:

### 1. Zusammensetzung für Triebfermente geeignet für Sauerteige in einstufiger Führung

Für die Zusammensetzungen der Triebfermente für Weizensauerteige werden die in Tabelle 6 nachfolgend aufgeführten Mengen an Mikroorganismen, die aus Reinkulturen isoliert wurden, vermengt. Die Mischung wird anschließend in Portionsgrößen mit ca. 10⁷-10⁹ KbE/g abgepackt.

**Tabelle 6**

| | Mikroorganismus | KbE/g |
|---|---|---|
| Triebferment A | *L. crispatus* | ca. 1 x 10⁸ |
| | *L. pontis* | ca. 1 x 10⁸ |
| | *L. plantarum* | ca. 1 x 10⁸ |
| | *L. sanfranciscensis* | ca. 1 x 10⁸ |
| | *S. cerevisiae* | ca. 1 x 10⁸ |
| | | |
| Triebferment B | *P. pentosaceus* | ca. 1 x 10⁹ |
| | *W. cibaria* | ca. 1 x 10⁸ |
| | *W. confusa* | ca. 1 x 10⁸ |
| | *S. cerevisiae* | ca. 1 x 10⁷ |
| | | |
| Triebferment C | *L. plantarum* | ca. 1 x 10⁸ |
| | *L. frumenti* | ca. 1 x 10⁷ |
| | *L. paracasei* | ca. 1 x 10⁷ |
| | *Le. argentinum* | ca. 1 x 10⁸ |
| | *L. helveticus* | ca. 1 x 10⁷ |
| | *L. paralimentarius* | ca. 1 x 10⁷ |
| | *L. fermentum* | ca. 1 x 10⁸ |
| | *S. pastorianus* | ca. 1 x 10⁷ |
| | *P. pentosaceus* | ca. 1 x 10⁷ |
| | | |
| | | |
| Triebferment D | *L. sanfranciscensis* | ca. 1 x 10⁹ |
| | *C. humilis* | ca. 1 x 10⁷ |
| | *L. suntoryeus* | ca. 1 x 10⁸ |
| | *L. pontis* | ca. 1 x 10⁸ |
| | *L. crispatus* | ca. 1 x 10⁸ |
| | *S. cerevisiae* | ca. 1 x 10⁷ |

### 2. Einstufige Vorteigführung mit Triebferment A oder B

Für das Erstellen eines Weizenvorteiges werden 30 kg Triebferment mit 30 kg Weizenmehl (Typ 550) und 301 Wasser vermengt. Die Anfangstemperatur der Mischung sollte 22-24°C haben. Da der Ansatz sehr gärintensiv ist, ist darauf zu achten, dass die Ansatzgefäße mindestens ein doppeltes Gärvolumen fassen können. Nach einer Stehzeit von mindestens 8 Stunden bei Raumtemperatur ist der Vorteig fertig und kann weiter verarbeitet werden bzw. bei 4-8°C gelagert werden. Die Verarbeitungstoleranz nach dem Ansetzen, ist etwa 8-24 Stunden. Sofern es erwünscht ist, kann von dem Vorteig ein Anstellgut für den nächsten Tag abgenommen werden. Vorzugsweise werden 30 kg Anstellgut abgenommen. Das Anstellgut wird bis zur Weiterverwendung bei 4-8°C gelagert.

Für das Backen ohne Hefe sollte 30-40% des Mehls fermentiert werden. Daher empfiehlt es sich für eine Teigrezeptur mit insgesamt 100 kg, 70 kg Weizenmehl (Typ 550) mit 60 kg Weizenvorteig entsprechend der oben aufgeführten Vermehrungsstufe anzusetzen.
Alternativ kann auch eine Teigrezeptur mit 40% fermentierten Mehl auf eine Gesamtmehlmenge von 100 kg angesetzt werden. Dazu werden 60 kg Weizenmehl (Typ 550) mit 80 kg Weizenvorteig, wie oben angesetzt, bereit gestellt. Die Teigtemperatur beträgt idealerweise zwischen 26 und 28°C. Der Teig ruht bzw. gärt für ca. 1-1,5 Stunden ggf. bis zu 3 Stunden, bevor er ausgebacken wird.

### 3. Einstufige Vorteigführung zur Erzeugung eines Weizenmischbrotes mit Triebferment A oder B

Für eine 10 kg Gesamtmehlmenge wird zunächst 2,8 kg Triebferment A oder B mit 2,8 kg Weizenmehl (Typ 550) und ca. 2,8 1 Wasser zu einem Vorteig angesetzt. Die Teigtemperatur beträgt zwischen 18 und 24°C. Nach ca. 6 Stunden ist der Vorteig fertig und es kann ggf. Anstellgut abgenommen werden, welches bis zur weiteren Verarbeitung kühl zu stellen ist. Nach 8 Stunden ist der Teig kühl zu stellen bzw. zu verarbeiten. Die Verarbeitungstoleranz beträgt bis zu 36 Stunden.
Für den Brotteig werden 5,6 kg des Vorteiges mit 4,2 kg Weizenmehl (Typ 550) und 3 kg Roggenmehl sowie 41 Wasser und 0,2 kg Salz vermengt. Idealerweise wird der Teig in einem Spiralkneter für 3+3 Minuten geknetet. Die Teigtemperatur beträgt zwischen 23 und 28°C. Nach einer 60-minütigen Teigruhe bei 32°C wird der Teig in Stücke zu je 750 gr. abgewogen und nach 80-90 Minuten bei 32°C Stückgare für 40-50 Minuten bei 250°C abfallend auf 210°C ausgebacken.

### 4. Einstufige Vorteigführung für Baguette ohne Backhefe mit Triebferment A oder B

Für eine Gesamtmehlmenge von 10 kg wird zunächst 3 kg Triebferment A oder B mit 3 kg Weizenmehl (Typ 550) und ca. 31 Wasser vermengt. Die Teigtemperatur beträgt zwischen 20 und 26°C. Nach ca. 6 Stunden kann von dem reifen Vorteig Anstellgut abgenommen werden, welches bei 4-8°C bis zur Verarbeitung kühl zu stellen ist. Der reife Vorteig wird für ca. 8 Stunden kühl gestellt, woran sich eine bis zu 36 Stunden anhaltende Verarbeitungszeit anschließt. Für den Baguetteteig werden 6 kg Vorteig mit 7 kg Weizenmehl und ca. 3,2 1 Wasser sowie 0,2 kg Salz vermengt. Idealerweise wird der Teig in einem Spiralkneter 3+3 Minuten geknetet. Die Teigtemperatur beträgt zwischen 20 und 24°C. Der Teig sollte vorsichtig bearbeitet werden, um die typische Porung zu erzielen. Die Gare findet außerhalb des Gärraumes statt, da dort die Luft zu feucht ist und zu hohe Temperaturen herrschen. Während der 60-minütigen Teigruhe wird der Teig nach ca. 30 Minuten einmal aufgezogen (leicht einschlagen). Es werden Teigstücke von 300 gr. abgewogen und in Form gebracht. Damit der Teig etwas mehr Stabilität bekommt, ruht er für weitere 10 Minuten. Danach in Baguetteform mit dünnen Spitzen kurz lang rollen und in Tücher einziehen. 90 Minuten auf Gare stellen. Dann auf Abzieher setzen und mit einer scharfen Klinge/Messer einschneiden. Die Teiglinge mit Vorschwaden in den Ofen bringen und ca. 30-35 Minuten bei 230°C ausbacken.

### 5. Einstufige Vorteigführung mit Triebferment A oder B für Panettone

Für eine Gesamtmehlmenge von 10 kg wird zunächst 4 kg Triebferment A oder B mit 4 kg Weizenmehl (Typ 550) und ca. 41 Wasser für 6 Stunden bei 22-24°C fermentiert. Nach 6 Stunden kann vom reifen Vorteig Anstellgut abgenommen werden, welches bis zur weiteren Verarbeitung kühl gelagert werden muss. Der Vorteig ist für weitere 6 Stunden kühl zu stellen, danach ist er bis zu 36 Stunden verarbeitungsfähig. Für den Panettoneteig werden 8 kg Vorteig mit 6 kg Weizenmehl (Typ 550), 1,6 kg Zucker, 1,6 kg Eier, 2,5 kg Butter, 0,1 kg Salz, 1,6 kg Früchten (Sultaninen, Orangeat, Zitronat) vermengt. Idealerweise wird der Teig im Spiralkneter 4+6 Minuten geknetet. Die Teigtemperatur beträgt 28°C. Nach einer 60-minütigen Teigruhe wird der Teig schonend abgewogen, leicht rund gewickelt und in die Panettoneform gelegt. Der Teig wird anschließend auf Gare gestellt, wobei die Gärzeit 3-4 Stunden im Gärraum bei 30-32°C beträgt. Wenn der Teig in der Form zu ¾ gegangen ist, wird mit einer Schere über Kreuz eingeschnitten und die Form mit Schwaden in die Ofen gebracht. Die Backzeit beträgt 50 Minuten, bei 200°C fallend auf 180°C. Im Anschluss an den Backvorgang wird der Panettone mit Butter bestrichen und über Kopf hängend ausgekühlt.

### 6. Einstufige Führung mit Triebferment A, B, oder D für mildes Roggenbrot

Für eine 10 kg Gesamtmehlmenge wird zunächst 2,8 kg Triebferment A oder B mit 2,8 kg Roggenmehl (Typ 997) und ca. 2,8 1 Wasser zu einem Vorteig angesetzt. Die Teigtemperatur beträgt zwischen 20 und 24°C. Nach ca. 6 Stunden ist der Vorteig fertig und es kann ggf. Anstellgut abgenommen werden, welches bis zur weiteren Verarbeitung kühl zu stellen ist. Die Verarbeitungstoleranz beträgt bis zu 24 Stunden.
Für den Brotteig werden 5,6 kg des Vorteiges mit 7,2 kg Roggenmehl (Typ 997) sowie 5 l Wasser und 0,2 kg Salz vermengt. Idealerweise wird der Teig in einem Spiralkneter für 6 Minuten langsam geknetet. Die Teigtemperatur beträgt zwischen 26 und 28°C. Nach einer 60-minütigen Teigruhe bei 32°C wird der Teig in Stücke zu je 850 gr. abgewogen und nach 80-90 Minuten bei 32°C Stückgare für 40-50 Minuten bei 250°C abfallend auf 210°C ausgebacken.

### 7. Einstufige Führung mit Triebferment C zur Herstellung glutenfreier Backwaren

Zunächst wird 40 g Triebferment C mit 200 g Reismehl und ca. 200 ml Wasser für 15-18 Stunden bei 25-27°C fermentiert. Nach 8 Stunden kann vom reifen Vorteig Anstellgut abgenommen werden, welches bis zur weiteren Verarbeitung kühl gelagert werden muss. Für den glutenfreien Brotteig werden 400 g Vorteig mit 500g Teffmehl, 250 g Buchweizenmehl, 250 g Maismehl, 20 g Salz, 30 g Guarkernmehl und 1100 ml Wasser vermengt. Idealerweise wird der Teig im Spiralkneter 5 Minuten geknetet. Die Teigtemperatur beträgt 28°C. Nach einer 10-minütigen Teigruhe wird der Teig in Kästen eingewogen. Der Teig wird anschließend auf Gare gestellt, wobei die Gärzeit 1,5-3 Stunden im Gärraum bei 30-32°C beträgt. Die Backzeit beträgt 60 Minuten, bei 200°C .

### 8. Einstufige Führung mit Triebferment A oder B zur Croissantherstellung

Für eine Gesamtmehlmenge von 10 kg wird zunächst 4 kg Triebferment A oder B mit 4 kg Weizenmehl (Typ 550) und ca. 41 Wasser vermengt. Die Teigtemperatur beträgt zwischen 20 und 22°C. Nach ca. 4-6 Stunden kann von dem reifen Vorteig Anstellgut abgenommen werden, welches bei 4-8°C bis zur Verarbeitung kühl zu stellen ist. Für den Croissantteig werden 8 kg Vorteig mit 6 kg Weizenmehl, 0,5 kg Zucker, 0,2 kg Butter und ca. 1 l Wasser sowie . Idealerweise wird der Teig in einem Spiralkneter 2+5 Minuten geknetet. Die Teigtemperatur beträgt zwischen 25 und 26°C. Teigstücke von 4,000 kg abwiegen (direkt nach der Knetung) und 30 Minuten abgedeckt im Kühlhaus entspannen lassen. Nach der Teigruhe 4,000 kg Teig und 1,000 kg Butter 2 einfache Touren geben. Danach den tourierten Teig 20 Minuten abgedeckt im Kühlhaus entspannen lassen, nochmals 1 einfache Tour geben und wiederum 20 Minuten kühlen. Danach wie gewohnt aufarbeiten. 2-3 Sunden bei max. 28 °C auf Gare stellen. Bei 200 °C (Ladenbackofen) 15-20 min. backen

### 9. Einstufige Vorteigführung mit Triebferment A oder B für Pandoro

Für eine Gesamtmehlmenge von 10 kg wird zunächst 3 kg Triebferment A oder B mit 3 kg Weizenmehl (Typ 550) und ca. 3 1 Wasser für 4 - 6 Stunden bei 22-24°C fermentiert. Nach 6 Stunden kann vom reifen Vorteig Anstellgut abgenommen werden, welches bis zur weiteren Verarbeitung kühl gelagert werden muss. Für den Pandoroteig werden 6 kg Vorteig mit 7 kg Weizenmehl (Typ 550), 2 kg Zucker, 1,6 kg Butter, 1,4 l Milch, 1,0 kg Eier, 0,8 kg Eigelb, Salz, Zitronenschale und Vanillestange vermengt. Idealerweise wird der Teig im Spiralkneter 4+6 Minuten geknetet. Die Teigtemperatur beträgt 26-28°C. Nach einer 60-minütigen Teigruhe wird der Teig in die Pandoroform gelegt. Der Teig wird anschließend auf Gare gestellt, wobei die Gärzeit 3-4 Stunden im Gärraum bei 30-32°C beträgt. Die Backzeit beträgt 60-75 Minuten, bei 180°C. Im Anschluss an den Backvorgang wird der fertige Pandoro sofort aus der Form gestürzt und mit Puderzucker betäubt.

## Patentansprüche

1. Triebferment **dadurch gekennzeichnet, dass**
- es frei von Backhefe ist;
- es eine Mischung aus wenigstens zwei kultivierten Reinkulturen von Milchsäurebakterien, ausgewählt aus der Gruppe bestehend aus *L. plantarum, L. pontis, L. sanfranciscensis, L. crispatus, L. suntoryeus, Le. argentinum, L. helveticus, L. paralimentarius, L. fermentum, L. paracasei, L. frumenti, L. alimentarius, W cibaria, W confusa, P. acidilactici, P. parvulus* und *P. pentosaceus,* wobei wenigstens eine ausgewählte Reinkultur ein Pediokokken Stamm ist, enthält; und
- es wenigstens einen kultivierten Stamm von Hefesprosspilzen enthält, ausgewählt aus der Gruppe bestehend aus *C. humilis, C. milleri, S. exiguus, S. cerevisiae, S. minor, S. pastorianus* und *S*. *fructuum,* wobei die besagten Hefesprosspilze zu keinem Zeitpunkt auf Melasse gezüchtet oder kultiviert wurden.

2. Triebferment nach Anspruch 1 zur Herstellung von Backwarenerzeugnissen **dadurch**
**gekennzeichnet, dass**
bei der Vorteig- oder Teigproduktion sich bei direkter Führung nach 5 bis 24 Stunden Inkubationszeit einen Milchsäuregehalt von maximal 1% erreicht wird.

3. Triebferment nach Anspruch 1 oder 2 zur Herstellung von Backwarenerzeugnissen
**dadurch gekennzeichnet, dass**
bei der Vorteig- oder Teigproduktion sich bei direkter Führung nach 5 bis 24 Stunden Inkubationszeit ein Gärungsquotient von 1,0 bis 10 einstellt.

4. Triebferment nach Anspruch 1 oder 2 zur Herstellung von Backwarenerzeugnissen
**dadurch gekennzeichnet, dass**
der Vorteig- oder die Teigproduktion bei direkter Führung nach 5 bis 24 Stunden Inkubationszeit einen Maltosegehalt von 0,3 bis 1,8 % hat.

5. Triebferment nach einem der Ansprüche 1 bis 4 zur Herstellung von Backwarenerzeugnissen **dadurch gekennzeichnet, dass**
der erzeugte Vorteig- oder die Teigproduktion nach 5 bis 24 Stunden Inkubationszeit eine CO2 Produktionsrate von 70 bis 300 ml/100g Mehl aufweist.

6. Sauerteigbrot hergestellt mit dem Triebferment nach einem der Ansprüche 1 bis 5 **dadurch gekennzeichnet, dass** die Krume des fertig gebackenen Brotes den Aromastoff Vanillin in einer Konzentration von größer 1000 µg/kg Trockenmasse enthält.

7. Sauerteigbackwaren hergestellt mit dem Triebferment nach einem der Ansprüche 1 bis 5 **dadurch gekennzeichnet, dass** die Backware oder das Brot einen Maltosegehalt von 0,3 bis 1,8 % aufweist.

8. Verfahren zur Herstellung von Backwaren aus Sauerteig **dadurch gekennzeichnet, dass**
- ein einem ersten Schritt Mehl mit Wasser und einem Triebferment angesetzt wird und diese Mischung in direkter Führung bei 15° bis 30°C inkubiert wird;
- in einem zweiten Schritt weitere Backzutaten und Inhaltsstoffe, jedoch keine Backhefe oder andere Triebmittel zugegeben werden;
- die Backware in einem dritten Schritt nach einer Ruhephase von 1 bis 3 Stunden ausgebacken wird; und
- die erzeugte Backware einen Milchsäuregehalt von maximal 1% aufweist.

9. Verfahren nach Anspruch 8, wobei das Triebferment frei von Backhefe ist, eine Mischung aus wenigstens zwei kultivierten Reinkulturen von Milchsäurebakterien enthält, ausgewählt aus der Gruppe bestehend aus *L. plantarum, L. pontis, L. sanfranciscensis, L. crispatus, L. suntoryeus, Le. argentinum, L. helveticus, L. paralimentarius, L. fermentum, L. paracasei, L. frumenti, L. alimentarius, W cibaria, W cortfusa, P. acidilactici, P. parvulus* und *P. pentosaceus,* und wenigstens einen kultivierten Stamm von Hefesprosspilzen enthält, ausgewählt aus der Gruppe bestehend aus *C. humilis, C. milleri, S. exiguus, S. cerevisiae, S. minor, S. pastorianus* und *S*. *fructuum,* wobei die besagten Hefesprosspilze zu keinem Zeitpunkt auf Melasse gezüchtet oder kultiviert wurden.

10. Verfahren nach Anspruch 8 oder 9, wobei sich durch den Einsatz des Triebferments bei der Vorteig- oder Teigproduktion in direkter Führung nach 5 bis 24 Stunden ein Gärungsquotient von 1,0 bis 10 einstellt.

11. Verfahren nach einem der Ansprüche 8 bis 10, wobei der Vorteig oder Teig nach 16 bis 24 Stunden Inkubationszeit eine CO2 Produktionsrate von 70 bis 300 ml/100g Mehl aufweist.

12. Verfahren nach einem der Ansprüche 8 bis 11, wobei hydrolytische Enzyme zur Intensivierung der Geschmacksentwicklung zugegeben werden.

13. Sauerteigbrot hergestellt nach dem Verfahren nach einem der Ansprüche 8 bis 12 **dadurch gekennzeichnet, dass** die Krume des fertig gebackene Brot den Aromastoff Vanillin in einer Konzentration von größer 1000 µg/kg Trockenmasse enthält.

14. Sauerteigbackwaren hergestellt nach dem Verfahren nach einem der Ansprüche 8 bis 12 **dadurch gekennzeichnet, dass** die Backware oder das Brot einen Maltosegehalt von 0,3 bis 1,8 % aufweist.

15. Verfahren zur Herstellung von Backwaren nach Anspruch 8 oder 9, wobei sich durch den Einsatz des Triebferments bei der Vorteig- oder Teigproduktion in direkter Führung der Einsatz von kommerzieller Backhefe reduzieren lässt.
